# EUROPEAN PATENT SPECIFICATION

(11) **EP 3 958 887 B1**
(45) Date of publication and mention of the grant of the patent: **01.11.2023**
(21) Application number: 20720445.4
(22) Date of filing: 22.04.2020
(51) Int. Cl.: A61K 38/20, A61K 31/00, A61K 35/17, A61P 37/02

(54) **MEDICAL USES FOR INDUCING OR RESTORING IMMUNE TOLERANCE**
MEDIZINISCHE VERFAHREN ZUR INDUKTION ODER WIEDERHERSTELLUNG DER IMMUNTOLERANZ
PROCÉDURES MÉDICALES D'INDUCTION OU DE RÉCUPÉRATION DE LA TOLÉRANCE IMMUNITAIRE

(30) Priority: 23.04.2019 EP 19305520
(43) Date of publication of application: 02.03.2022
(73) Proprietor: Institut National de la Santé et de la Recherche Médicale (INSERM), 75013 Paris (FR); Université Paris Cité, 75006 Paris (FR); Fondation Imagine, 75015 Paris (FR); Assistance Publique-Hôpitaux de Paris (APHP), 75004 Paris (FR)
(72) Inventor: ANDRE, Isabelle, 75015 Paris (FR); ZUBER, Julien, 75015 Paris (FR); SIX, Emmanuelle, 75015 Paris (FR); DELVILLE, Marianne, 75015 Paris (FR); CAVAZZANA, Marina, 75015 Paris (FR)
(74) Representative: Inserm Transfert
(86) International application number: PCT/EP2020/061254
(87) International publication number: WO 2020/216807

(56) References cited:
- WO-A1-2009/045464
- WO-A1-2014/180943
- WO-A1-2019/012024
- WO-A1-2019/040655
- WO-A2-2007/084775
- US-A1- 2015 374 788

## Description

### FIELD:

The present disclosure is in the field of autoimmunity.

### BACKGROUND:

The global frequency of autoimmune diseases is between 3 and 5% in developed countries and has continuously increased in the last years. More than 100 different autoimmune diseases have been reported, which all correspond to chronic diseases triggered by a loss of immune tolerance against self-antigens. The most frequent or described ones are rheumatoid arthritis, systemic lupus erythematous, due to the production of antibodies directed against self-antigens, inflammatory bowel disease, multiple sclerosis and type 1 diabetes due to aberrant T cell responses. Most have a multifactorial origin involving both genetic (among which polymorphisms in HLA loci), endogenous (chronic inflammation, hormones) and environment (stress, nutrition, viral infections, anti-cancer treatments) factors, as well as diverse targeted organs. Some rare and very severe autoimmune conditions are of hereditary origin such as APECED and IPEX syndrome due to altered negative selection of autoreactive T cells in the thymus or absence of regulatory T cells (Treg). Treatments include replacement therapy (for instance insulin treatment), corticoids, immunosuppressive treatments, immunotherapies (anti-cytokine treatments), and for the most severe cases, autologous or allogenic hematopoietic stem cell transplantation. Adoptive transfer of Treg are also suitable for the treatment of autoimmune diseases, such as diabetes (Tang, Q. J. Exp. Med. 199, 1455-1465, 2004) or inflammatory bowel disease (Mottet, C., J. Immunol. Baltim. Md 1950 170, 3939-3943, 2003). Adoptive transfer of healthy CD4+CD25++ Treg (4×10⁵ cells) in neonatal (1 or 2 days old) scurfy mice (a murine model of IPEX syndrome) is enough to prevent the development of the disease (Fontenot, J. D., Nat. Immunol. 4, 330-336 (2003).39. Mottet, C., Uhlig, H. H. & Powrie, F. Cutting edge: cure of colitis by CD4+CD25+ regulatory T cells. J. Immunol. Baltim. Md 1950 170, 3939-3943 (2003)). Moreover, transgenic restoration of FoxP3 expression prevents scurfy disease in FoxP3sf/Y mice (Brunkow ME Nat Genet 2001). However, up to date, all studies focused on the prevention and not on the treatment of the disease, which constitutes the final aim of any clinical application. So, there is a need for new methods for the treatment of autoimmunity.

Donor-specific tolerance has long been the Holy Grail in transplantation, with the ultimate goal to avoid life-threatening complications of long-term immunosuppression. Combined kidney and bone marrow transplantation (CKBMT) has been successfully used to induce immune tolerance through mixed chimerism, defined as a state wherein donor and recipient hematopoietic cells coexist. This strategy has offered proof of concept that operational tolerance can be induced in humans. However, this success came at heavy toll due to harsh cytoreductive regimens and donor lymphocyte infusion with ensuing severe complications, including fatal graft-versus-host disease (GVHD).

Hence, there is room to improve the current tolerogenic protocols by promoting peripheral tolerance mechanisms. FOXP3-expressing regulatory T cells (hereinafter referred to as Tregs) are key peripheral regulators of the immune system and are mandatory for preventing autoimmune diseases. Mounting evidence implicates Tregs in clinical and experimental transplant tolerance induction. A significant expansion of donor-specific Tregs was found at 6 months after CKBMT in tolerant patients, unlike in the nontolerant patients. Moreover, the administration of donor-specific Tregs-enriched cell product allowed successful weaning and cessation of immunosuppressive agents in seven out of ten liver transplant recipients (Todo, Satoru, et al. "A pilot study of operational tolerance with a regulatory T - cell - based cell therapy in living donor liver transplantation." Hepatology 64.2 (2016): 632-643). Thus, adoptive Treg therapy holds promise as an alternative to immunosuppressive drugs. However, Treg cellular therapy in transplantation faces 3 main challenges, including their isolation and expansion, the very low frequency of donor-specific Tregs, and the high number of cells required to outcompete alloreactive effector T cells (Teffs). In this respect, pilot studies suggest that Treg activation through CD28-CAR-signaling preserves suppressive function. CAR-Tregs have demonstrated a far greater efficiency than polyclonal Tregs in controlling rejection and Graft-vs-Host Disease (GVHD) in transplant models.

Post-transplant cyclosphosphamide pulse was found very efficient at shrinking in size the alloimmune response in both bone marrow (Robinson, Tara M., et al. "Haploidentical bone marrow and stem cell transplantation: experience with post-transplantation cyclophosphamide." Seminars in hematology. Vol. 53. No. 2. WB Saunders, 2016) and solid organ (Todo, Satoru, et al. "A pilot study of operational tolerance with a regulatory T - cell - based cell therapy in living donor liver transplantation." Hepatology 64.2 (2016): 632-643.) transplantations and was successfully combined with donor-specific Tregs in order to promote tolerance (Todo, Satoru, et al. "A pilot study of operational tolerance with a regulatory T - cell - based cell therapy in living donor liver transplantation." Hepatology 64.2 (2016): 632-643.).

WO2014/180943 discloses a vector containing nucleic acid encoding Foxp3 and Mcl-1 or containing nucleic acid encoding Foxp3 and/or Mcl-1 operatively linked to a Foxp3 promoter, in particular for use in the treatment of disease characterized by Treg deficiency.

WO2019/040655 discloses lentiviral vectors expressing Foxp3 in hematopoietic stem cells to treat immune deficiencies and autoimmune diseases.

WO2019/012024 discloses methods for increasing expansion and immunosuppressive capacity of a population of CD8+CD45RC^{low/-} Tregs.

### SUMMARY:

The present invention is defined by the claims.

In particular, the present invention relates to an amount of cyclophosphamide, an amount of a population of Treg cells and an amount of a IL-2 polypeptide for use in a method of inducing or restoring immune tolerance in a patient suffering from IPEX syndrome comprising the steps of i) administering the patient with the said amount of cyclophosphamide, ii) then engrafting the patient with the said amount of the population of Treg cells, and iii) finally administering the patient with the said amount of a IL-2 polypeptide.

The following detailed description, figures and example do not fall under the scope of the present invention and are present for understanding and illustration purposes only. Furthermore, any reference in the description to methods of treatment refers to the compounds, pharmaceutical compositions and medicaments of the present invention for use in a method of treatment of the human body by therapy.

### DETAILED DESCRIPTION:

Some rare and very severe autoimmune conditions are of hereditary origin such as APECED and IPEX syndrome due to altered negative selection of autoreactive T cells in the thymus or absence of regulatory T cells (Treg). Innovative strategies based on the use of regulatory T cells have been developed. We have now compared 7 different experimental protocols to identify the one allowing to get the most efficacy of Treg to treat Scurfy autoimmune syndrome, a severe autoimmune model mimicking IPEX syndrome. The optimized protocol comprised a preconditioning step using cyclophosphamide and a post-conditioning step using IL-2.

Thus, the first object herein disclosed relates to an amount of cyclophosphamide, an amount of a population of Treg cells and an amount of a IL-2 polypeptide for use in a method of inducing or restoring immune tolerance in a patient suffering from IPEX syndrome comprising the steps of i) administering the patient with the said amount of cyclophosphamide, ii) then engrafting the patient with the said amount of the population of Treg cells, and iii) finally administering the patient with the said amount of a IL-2 polypeptide.

As used herein, the term "immune tolerance" refers to a state of unresponsiveness of the immune system to specific substances or tissues that have the capacity to elicit an immune response while preserving immune responses against other substances or tissues. As used herein, the term "immune response" includes T cell mediated and/or B cell mediated immune responses. Exemplary immune responses include T cell responses, e.g., cytokine production and cellular cytotoxicity, in addition, the term immune response includes immune responses that are indirectly affected by T cell activation, e.g., antibody production (humoral responses) and activation of cytokine responsive cells, e.g., macrophages. Immune cells involved in the immune response include lymphocytes, such as B cells and T cells (CD4+, CD8+, Thl and Th2 cells); antigen presenting cells (e.g. professional antigen presenting cells such as dendritic cells); natural killer cells; myeloid cells, such as macrophages, eosinophils, mast cells, basophils, and granulocytes.

As used herein, the term "IPEX syndrome" has its general meaning in the art and a disease that results in most cases from mutations in FoxP3. IPEX syndrome usually develops during the first few days or weeks of life and affects exclusively boys. It manifests with the sequential appearance of the triad of enteropathy, autoimmune endocrinopathies, and cutaneous involvement, but the clinical features and severity of the disease can vary considerably between individuals. Severe autoimmune enteropathy manifests with intractable secretory diarrhea leading to malabsorption, electrolyte disturbance and failure to thrive. Vomiting, ileus, gastritis or colitis can also be observed. Patients also present with autoimmune endocrinopathies, generally insulin-dependent diabetes mellitus (type 1 DM), but also thryroiditis leading to hypothyroidism or hyperthyroidism. Skin involvement consists of a generalized pruriginous eruption resembling eczema, psoriasis, and/or atopic or exfoliative dermatitis. Less frequently, alopecia or onychodystrophy can be observed. Patients may develop autoimmune cytopenias, thrombocytopenia, hemolytic anemia and neutropenia. Autoimmune involvement may also lead to pneumonitis, hepatitis, nephritis, myositis, splenomegaly and/or lymphadenopathy. Local or systemic infections (e.g. pneumonia, *Staphylococcus aureus* infections, candidiasis) may occur but seem to be due to loss of skin and gut barriers, immunosuppressive therapies, and poor nutrition rather than a primary immunodeficiency. IPEX syndrome is caused by mutations in the FOXP3 gene (Xp11.23). More than 20 mutations of FOXP3 are reported in IPEX, and the syndrome is lethal if untreated. Diagnosis is based on clinical examination, family history, and laboratory findings revealing autoimmune enteropathy (anti-enterocyte, harmonin and villin autoantibodies), type 1 DM (antibodies against insulin, pancreatic islet cells, or anti-glutamate decarboxylase), thyroiditis (anti-thyroglobulin and anti-microsome peroxidase antibodies) and cytopenia (anti-platelets and anti-neutrophils antibodies, positive Coombs test). Molecular genetic testing confirms the diagnosis.

As used herein, the term "treatment" or "treat" refer to both prophylactic or preventive treatment as well as curative or disease modifying treatment, including treatment of patient at risk of contracting the disease or suspected to have contracted the disease as well as patients who are ill or have been diagnosed as suffering from a disease or medical condition, and includes suppression of clinical relapse. The treatment may be administered to a patient having a medical disorder or who ultimately may acquire the disorder, in order to prevent, cure, delay the onset of, reduce the severity of, or ameliorate one or more symptoms of a disorder or recurring disorder, or in order to prolong the survival of a patient beyond that expected in the absence of such treatment. By a "therapeutically effective amount" is meant a sufficient amount of cells generated with the present disclosure for the treatment of the disease at a reasonable benefit/risk ratio applicable to any medical treatment. It will be understood that the total usage of these cells will be decided by the attending physicians within the scope of sound medical judgment. The specific therapeutically effective dose level for any particular patient will depend upon a variety of factors including the age, body weight, general health, sex and diet of the patient; the time of administration, route of administration, and survival rate of the cells employed; the duration of the treatment; drugs used in combination or coincidental with the administered cells; and like factors well known in the medical arts. For example, it is well known within the skill of the art to start doses of cells at levels lower than those required to achieve the desired therapeutic effect and to gradually increase the dosage until the desired effect is achieved.

### Step i): administering an amount of cyclophosphamide:

In particular, the term "cyclophosphamide" has its general meaning in the art and refers to the generic name for 2-[bis(2-chloroethyl)amino]-tetrahydro-2H-1,3,2-oxazaphosphorine-2-oxide monohydrate.

We have found that an amount of cyclophosphamide of between 40 à 200 mg/m² may be used. In particular, an amount of about 40, 50, 60, 70, 80, 90, 100, 110, 120, 130, 140, 150, 160, 170, 180, 190 or 200 mg/m² may be used. Preferably an amount of 150 mg/m² is used.

As used herein, the term "about," as applied to one or more values of interest, refers to a value that is similar to a stated reference value. In particular, the term "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction of the stated reference value unless otherwise stated or otherwise evident from the context.

In particular, the amount of cyclophosphamide is administered to the patient in one bolus 2, 3, 4, 5, 6, 7, 8, 9 or 10 days before engrafting the patient with the amount of Treg cells. Preferably, the amount of cyclophosphamide is administered to the patient 4 days before the engraftment.

### Step ii): engrafting an amount of Treg cells

As used herein, the term "T cell" refers to a type of lymphocytes that play an important role in cell-mediated immunity and are distinguished from other lymphocytes, such as B cells, by the presence of a T-cell receptor on the cell surface.

As used herein, the term "regulatory T cells" or "Treg cells" refers to cells that suppress, inhibit or prevent T cells activity. As used herein, Treg cells have the following phenotype at rest CD4+CD25+FoxP3+ and thus are characterized by the expression of FoxP3.

As used herein, the term "T-cell progenitors" refers to progenitors of the T cells that migrate to and colonize the thymus. The developing progenitors within the thymus, also known as thymocytes, undergo a series of maturation steps that can be identified based on the expression of different cell surface markers. The majority of cells in the thymus give rise to αβ T cells.

As used herein, the term "FoxP3" has its general meaning in the art and refers to a transcription factor belonging to the forkhead/winged-helix family of transcriptional regulators. FOXP3 appears to function as a master regulator (transcription factor) in the development and function of regulatory T cells. FoxP3 confers T cells with regulatory function and increases the expression of CTLA-4 and CD25, but decreases IL-2 production by acting as a transcriptional repressor. FoxP3 binds to and suppresses nuclear factor of activated T cells (NFAT) and nuclear factor-kappaB (NFKB) (Bettelli, E.M. et al, 2005, Proc Natl Acad Sci U S A 102:5138).

In particular, the Tregs cells are prepared according to any well-known method in the art. In particular, the Treg cells are prepared by transfecting or transducing a population of T cells ex vivo with a vector comprising a nucleic acid encoding for FoxP3. Typically, the vector is a retroviral vector. As used herein, the term "retroviral vector" refers to a vector containing structural and functional genetic elements that are primarily derived from a retrovirus. In particular, the retroviral vector of the present disclosure derives from a retrovirus selected from the group consisting of alpharetroviruses (e.g., avian leukosis virus), betaretroviruses (e.g., mouse mammary tumor virus), gammaretroviruses (e.g., murine leukemia virus), deltaretroviruses (e.g., bovine leukemia virus), epsilonretroviruses (e.g., Walley dermal sarcoma virus), lentiviruses (e.g., HIV-1, HIV-2) and spumaviruses (e.g., human spumavirus). In particular, the retroviral vector of the present disclosure is a lentiviral vector. As used herein, the term "lentiviral vector" refers to a vector containing structural and functional genetic elements that are primarily derived from a lentivirus. In particular, the lentiviral vector of the present disclosure is selected from the group consisting of HIV-1, HIV-2, SIV, FIV, EIAV, BIV, VISNA and CAEV vectors. In particular, the lentiviral vector is a HIV-1 vector.

As used herein, the term "hematopoietic stem cells" (HSCs) refers pluripotent stem cells capable of self-renewal and that are characterized by their ability to give rise under permissive conditions to all cell types of the hematopoietic system. Hematopoietic stem cells are not totipotent cells, i.e. they are not capable of developing into a complete organism.

In particular, a gene editing approach for site- specific restoration of wild-type FOXP3 gene expression may be applied to T cells and/or hematopoietic stem cells (HSCs) and/or T-cell progenitors carrying FOXP3 mutations to correct Treg functional defects. As used herein, the term "gene editing approach" refers to a system comprising one or more DNA-binding domains or components and one or more DNA-modifying domains or components, or isolated nucleic acids, e.g., one or more vectors, encoding said DNA- binding and DNA-modifying domains or components. Gene editing systems are used for modifying the nucleic acid of a target gene and/or for modulating the expression of a target gene. In known gene editing systems, for example, the one or more DNA-binding domains or components are associated with the one or more DNA-modifying domains or components, such that the one or more DNA-binding domains target the one or more DNA-modifying domains or components to a specific nucleic acid site. Polypeptide components of a gene editing systems are referred to herein as "gene editing proteins." Gene editing systems are known in the art, and include but are not limited to, zinc finger nucleases, transcription activator-like effector nucleases (TALENs); clustered regularly interspaced short palindromic repeats (CRISPR)/Cas systems, and meganuclease systems.

Thus, in particular, T cells and/or hematopoietic stem cells (HSCs) and/or T-cell progenitors are contacted with a CRISPR-associated endonuclease and at least one guide RNA.

As used herein, the term "CRISPR-associated endonuclease" has its general meaning in the art and refers to clustered regularly interspaced short palindromic repeats associated which are the segments of prokaryotic DNA containing short repetitions of base sequences. In particular, the CRISPR-associated endonuclease is a Cas9 nuclease. In particular, the CRISPR-associated endonuclease is a Cpfl nuclease. As used herein, the term "Cpfl protein" to a Cpfl wild-type protein derived from Type V CRISPR-Cpf1 systems, modifications of Cpf1 proteins, variants of Cpfl proteins, Cpfl orthologs, and combinations thereof.

As used herein, the term "guide RNA" or "gRNA" has its general meaning in the art and refers to an RNA which can be specific for a target DNA and can form a complex with the CRISPR-associated endonuclease.

In particular, the CRISPR-associated endonuclease and the guide RNA are provided to the cells through expression from one or more expression vectors. In particular, the CRISPR endonuclease can be encoded by the same nucleic acid as the guide RNA sequences. Vectors can include, for example, viral vectors (such as adenoviruses ("Ad"), adeno- associated viruses (AAV), and vesicular stomatitis virus (VSV) and retroviruses), liposomes and other lipid-containing complexes, and other macromolecular complexes capable of mediating delivery of a polynucleotide to a host cell.

In particular, the CRISPR-associated endonuclease can be pre-complexed with a guide RNA to form a ribonucleoprotein (RNP) complex. As used herein, the term "ribonucleoprotein complex," or "ribonucleoprotein particle" refers to a complex or particle including a nucleoprotein and a ribonucleic acid. A "nucleoprotein" as provided herein refers to a protein capable of binding a nucleic acid (e.g., RNA, DNA). Where the nucleoprotein binds a ribonucleic acid, it is referred to as "ribonucleoprotein." The interaction between the ribonucleoprotein and the ribonucleic acid may be direct, e.g., by covalent bond, or indirect, e.g., by non-covalent bond (e.g. electrostatic interactions (e.g. ionic bond, hydrogen bond, halogen bond), van der Waals interactions (e.g. dipole-dipole, dipole-induced dipole, London dispersion), ring stacking (pi effects), hydrophobic interactions and the like). The RNP complex can thus be introduced into the cells. Typically, the RNP complex is produced simply by mixing Cas9 and one or more guide RNAs in an appropriate buffer. This mixture is incubated for 5-10 min at room temperature before electroporation.

In particular, the population of Treg cells and/or hematopoietic stem cells (HSCs), and/or T-cell progenitors is/are genetically modified to encode desired expression products, as will be further described below. The term "genetically modified" indicates that the cells comprise a nucleic acid molecule not naturally present in non-modified population of Treg cells and/or hematopoietic stem cells (HSCs), and/or T-cell progenitors or a nucleic acid molecule present in a non-natural state in said population of Treg cells and/or hematopoietic stem cells (HSCs) and/or T-cell progenitors(e.g., amplified). The nucleic acid molecule may have been introduced into said cells or into an ancestor thereof. A number of approaches can be used to genetically modify a population of cells, such as virus-mediated gene delivery, non-virus-mediated gene delivery, naked DNA, physical treatments, etc. To this end, the nucleic acid is usually incorporated into a vector, such as a recombinant virus, a plasmid, phage, episome, artificial chromosome, etc. Examples of means by which the nucleic acid carrying the gene may be introduced into the cells include, but are not limited to, microinjection, electroporation, transduction, or transfection using DEAE-dextran, lipofection, calcium phosphate or other procedures known to one skilled in the art.

The nucleic acid used to genetically modify the population of Treg cells and/or hematopoietic stem cells (HSCs) and/or T-cell progenitors may encode various biologically active products, including polypeptides (e.g., proteins, peptides, etc.), RNAs, etc. In particular, the nucleic acid encodes a polypeptide having an immuno-suppressive activity. Another preferred category of nucleic acids is those encoding a T cell and/or hematopoietic stem cells (HSCs) and/or T-cell progenitors receptor or a subunit or functional equivalent thereof such as a chimeric antigen receptor (CAR) specific to an antigen of interest or a chimeric autoantibody receptor (CAAR) comprising an auto-antigen. For instance, the expression of recombinant TCRs or CARs specific for an antigen produces human Treg cells and/or hematopoietic stem cells (HSCs) and/or T-cell progenitors, which can act more specifically and efficiently on effector T cells and/or hematopoietic stem cells (HSCs) and/or T-cell progenitors to inhibit immune responses in a patient in need thereof. The basic principles of chimeric antigen receptor (CAR) design have been extensively described (e.g. Sadelain et al., 2013). Thus, in particular, the Treg cells and/or hematopoietic stem cells (HSCs) and/or T-cell progenitors of the disclosure are genetically modified and express at least one CAR, one CAAR and/or one native receptor linked to intracellular signaling molecules. Examples of CAR included, without being limited to, first generation CARs, second generation CARs, third generation CARs, CARs comprising more than three signaling domains (co-stimulatory domains and activation domain), and inhibitory CARs (iCARs).

In particular, the population of Treg cells and/or hematopoietic stem cells (HSCs) and/or T-cell progenitorsis autologous to the patient, meaning the population of cells is derived from the same patient.

In particular, the Tregs cells and/or hematopoietic stem cells (HSCs) and/or T-cell progenitorsare formulated by first harvesting them from their culture medium, and then washing and concentrating the cells in a medium and container system suitable for administration (a "pharmaceutically acceptable" carrier) in a treatment-effective amount. Typically, the population of Tregs cells and/or hematopoietic stem cells (HSCs) and/or T-cell progenitors of the present disclosure is administered to the patient in the form of pharmaceutical composition. The pharmaceutical composition may be produced by those of skill, employing accepted principles of treatment. The pharmaceutical composition may be administered by any means that achieve their intended purpose. For example, administration may be by parenteral, subcutaneous, intravenous, intradermal, intramuscular, intraperitoneal, transdermal, or buccal routes. The pharmaceutical compositions may be administered parenterally by bolus injection or by gradual perfusion over time. The pharmaceutical compositions typically comprise suitable pharmaceutically acceptable carriers comprising excipients and auxiliaries which may facilitate processing of the active compounds into preparations which can be used pharmaceutically.

In particular, an amount of between 1×10⁶/kg and 10×10⁶/kg Treg cells is engrafted in the patient. In particular, an amount of 1×10⁶/kg, 2×10⁶/kg, 3×10⁶/kg, 4×10⁶/kg, 5×10⁶/kg, 6×10⁶/kg, 7×10⁶/kg, 8×10⁶/kg, 9×10⁶/kg, or 10×10⁶/kg Treg cells is engrafted in the patient. Preferably, an amount of about 5×10⁶/kg of Treg is engrafted in the patient.

In particular, the engraftment is performed in combination with another biologically active agent. As used herein, the term "biologically active agent" is an agent, or its pharmaceutically acceptable salt, or mixture of compounds, which has therapeutic, prophylactic, pharmacological, or physiological effects on a mammal. Typically, the biological agent is deemed to potentiate the immunosuppressive properties of the Tregs and/or hematopoietic stem cells (HSCs) and/or T-cell progenitors. The biological active agent may be selected from the group of (a) proteins or peptides, (b) nucleic acids and (c) organic or chemical substances.

### Step iii): administering an amount of an IL-2 polypeptide

As used herein, the term "IL-2" has its general meaning in the art and refers to the interleukin-2 that is typically required for T-cell proliferation and other activities crucial to regulation of the immune response. Thus, the term "IL-2 polypeptide" has its general meaning in the art and includes naturally occurring IL-2 and function conservative variants and modified forms thereof (i.e. "mutein"). The IL-2 can be from any source, but typically is a mammalian (e.g., human and non-human primate) IL-2, and more particularly a human IL-2. An exemplary human amino acid sequence for IL-2 is represented by SEQ ID NO:1. In particular, the IL-2 polypeptide is a IL-22 mutein that consists of the amino acid sequence as set forth in SEQ ID NO:1 wherein the residue (V) at position 91 is substituted by a reside (K). In particulars, the IL-2 polypeptide is AMG 592 that is IL-2 mutein designed for greater Treg selectivity and longer half-life compared with recombinant IL-2 (Tchao, Nadia, et al. "Amg 592 Is an Investigational IL-2 Mutein That Induces Highly Selective Expansion of Regulatory T Cells." (2017): 696-696).
SEQ ID NO:1 >sp|P60568|IL2_HUMAN Interleukin-2 OS=Homo sapiens OX=9606 GN=IL2 PE=1 SV=1

MYRMQLLSCIALSLALVTNSAPTSSSTKKTQLQLEHLLLDLQMILNGINNYKNPKLTRML
TFKFYMPKKATELKHLQCLEEELKPLEEVLNLAQSKNFHLRPRDLISNINVIVLELKGSE
TTFMCEYADETATIVEFLNRWITFCQSIISTLT

In particular, an amount of the IL-2 polypeptide of between 0,5 MUI (Million International Units) / day and 1,5 millions of MUI/day may be used. In particular, an amount of 0,5; 0,6; 0,7; 0,8; 0,9; 1; 1,1; 1,2; 1,3; 1,4; or 1,5 MUI/day is used. Preferably an amount of 1 MUI/day is used.

In particular, the amount of the IL-2 polypeptide is administered to the patient daily from day 1 to day 5 (the induction period) after engraftment, and then every 2 weeks from day 15 to day 180 (the maintenance period) after engraftment.

The disclosure will be further illustrated by the following figures and examples.

### FIGURES:

**Figure 1** **depicts the experiment 1.**
**Figure 2** **depicts the experiment 2.**
**Figure 3** **depicts the experiment 3.**
**Figure 4** **depicts the experiment 4.**
**Figure 5** **depicts the experiment 6.**
**Figure 6** **depicts the experiment 6.**
**Figure 7** **depicts the experiment** 7.

### MATERIAL AND METHODS

### Mice

Scurfy phenotype was obtained by backcrossing on B6.129S7-Rag1tm1Mom/J background, allowing generation of homozygous XSf/ XSf.Rag1-/- female. Crossing of these females with WT C57BL/6J mice result in the birth only of diseased XSf/Y.Rag1-/+ male.

### WT CD4 T cells

Splenocytes were harvested from C57BL/6J by aseptic removal. After gentle crushing of spleens through a 70 µM mesh filter, CD4+ T cells were isolated by negative selection using EasySep Mouse CD4+ T cell Isolation Kit (StemCell Technologies, Grenoble, France). Purity exceeded 90%.

### Scurfy CD4 T cells

From XSf/Y.Rag1-/+ mice of 10 days, lymph nodes were collected and CD4+ T cells were separated using Murine CD4+ T cell Isolation kit (Miltenyi Biotec, Paris, France). Briefly, CD4+ collected from lymph nodes were labeled with a cocktail of biotinylated antibodies targeting CD4- cells, followed by labeling with anti-biotin magnetic beads. Cells were separated on a LS column (Miltenyi Biotec) and CD4+ cells were collected in the flow through. Purity exceeded 90%.

### WT Tregs CD4+CD25+

Splenocytes and lymph nodes were harvested from B6LY5.1 CD45.1 (8-12 weeks) and CD4+ T cells were isolated using EasySep Mouse CD4+ T cell Isolation Kit. A staining of CD25+ cells was performed with an anti-CD25 PE antibody (clone PC61, BD Biosciences, Le Pont de Claix, France), and then CD4+CD25+ cells were sorted on SH800 (Sony Biotechnology, Weybridge, UK) or ARIA II (BD Biosciences) cells sorters with a nozzle of 100µm. For Treg suppression assay, CD4+CD25- cells were also sorted.

### Lentiviral vector

The cDNA for a truncated codon-optimized human ΔLNGFR and/or a codon optimized human FOXP3 was cloned in a pCCL backbone with different designs. Bidirectional vectors with the bidirectional promoters architecture, one allowing FOXP3 expression under the control of the ubiquitous elongation factor 1 alpha (EF1α) and ΔLNGFR under the control of phosphoglycerate kinase (PGK) human promoter and their mock counterpart containing only the ΔLNGFR reporter (LNGFRp-eFOXP3 and LNGFRp-e) and one allowing FOXP3 expression under the control of PKG and ΔLNGFR under the control of a short version of EF1α (EFS) LNGFRe-pFOXP3 and LNGFRe-p).

In T2A designs, expression is under the control of EF1α. Two constructs were built: ΔLNGFR followed by the T2A sequence and FOXP3 or FOXP3 followed by the T2A and ΔLNGFR.

### T cell Transduction

Freshly isolated CD4+ T cells were plated at 1.10⁶ cells/mL in round bottom plate in RPMI 1640 medium + GlutaMax (GIBCO, Thermo Fisher Scientific, Montigny-Le-Bretonneux, France) supplemented with 10% fetal bovine serum (GIBCO), 1% Penicillin-Streptomycin (GIBCO), 0,1% 2-mercaptoethanol (GIBCO). Medium was supplemented with recombinant murine IL-2 (Peprotech, Rocky Hill, USA) at a concentration of 100 UI/ml for WT CD4 T cells or 300 UI/ml for Scurfy CD4 T cells. Cells were activated and expanded with anti-CD3/CD28 Dynabeads (GIBCO) at a 1:1 bead:cell ratio. Transduction was performed according the protocol previously described ⁴³ (ref article LB). Briefly transduction medium (RPMI supplemented with 0,25mg/ml Lentiboost (Sirion Biotech, FlashTherapeutics, Toulouse, France)) was added to cells with lentiviral vector at a MOI 10 concomitantly with activation and incubated overnight. Transduced cells were stained at day 5 after transduction by ΔLNGFR PE antibodies (clone ME20.4-1.H4, Miltenyi Biotec) and sorted on SH800 (Sony Biotechnology).

### Adoptive T cells transfer

First, scurfy mice were treated with an immunosuppressor: Temsirolimus (LC laboratories, Woburn, USA) was injected S.C at the dose of 2 mg/kg at day 8 and day 10 after birth. This treatment was continued twice a week in some experiment. Anti-CD3 Fab'2 (clone 145-2C11, BioXCell, West Lebanon, USA) was injected S.C at 20µg/day during 5 days at day 8 after birth. Cyclophosphamide (European Pharmacopoeia (EP) Reference Standard, Merck KGaA, Darmstadt, Germany) was injected I.P. at 50, 100, or 150mg/kg 10 days afterbirth. At day 10 or day 14, CD4⁺CD25⁺ CD45.1⁺ cells (containing putative Tregs) or engineered CD4⁺ T cells (Foxp3.LNGFR or LNGFR) from Scurfy mice were injected at 0.5 ×10⁶, 0.75 ×10⁶ or 1×10⁶ of cells respectively via I.P injections. Vehicle (ie. PBS) was injected in the same volume IP for the mice that did not receive cells injection. Because of low titer of the mock LNGFRp-e vector, which hampered the production of high numbers of CD4^{LNGFR} cells, LNGFRe-p transduced Scurfy CD4 T cells were used to complete the group of CD4^{LNGFR} treated mice. In indicated experiments, together with cells, Proleukin (human IL-2, aldesleukine, Novartis) was injected at 1.000UI/g via I.P and during 5 days then once a week.

### Flow cytometry

Single cell suspensions from spleen and lymph nodes were obtained by gentle crushing of spleens through a 70 µM mesh filter. Samples from the lung and the liver were prepared after digestion with Collagenase IV (Thermo Fischer Scientific) followed by gentle crushing of spleens through a 100 µM mesh filter.

Samples were prepared for flow cytometry using the following method: Cells were resus-pended in 100 uL of FACS buffer (phosphate buffered saline (PBS, Corning) /2% Fetal Bovine Serum [GIBCO]) and incubated with 2 uL of each antibody and 7AAD (Miltenyi Biotec,) for 20-30 min at 4 C.

Cells were washed once in FACS buffer prior to analysis. For intracellular FoxP3 staining, cells were first stained with cell surface markers and fixable viability dye eF780 (eBioscience, Thermo Fischer Scientific) as described above. After washing, cells were fixed and permeabilized using the FoxP3 staining buffer set eBioscience, Thermo Fischer Scientific) according to manufacturers' directions. Human FoxP3-APC (eBioscience, Thermo Fischer Scientific) was added for 30-60 min at RT. Samples were acquired on a MACSquant flow cytometer (Miltenyi Biotec), BD LSR Fortessa cytometer (BD Biosciences) or a Sony Spectral SH6800 (Sony Biotechnology). Data were analyzed using FlowJo V10 (TreeStar). The following antibodies were used: anti-mouse CD62L APC-Cy7 clone MEL-14, CD44 APC clone IM7 (BD Biotechnology), CD45.1 APC-Cy7 clone A20, CD45.2 PeCy7 clone 104, CD134 clone OX-40 Brilliant Violet 421, CD279 (PD-1) clone 29F.1A12 Brilliant Violet 605, CD25 clone PC61 Brilliant Violet 711, TIGIT clone Vstm3 1G9 PE, CD357 (GITR) clone DTA-1 PerCP/Cy5.5, CD39 clone Duha59 PE/Cy7 and CD152 clone UC10-4B9 PE/Dazzle (Sony Biotechnology) and human ΔLNGFR PE clone ME20.4-1.H4 (Miltenyi Biotec), Helios clone 22F6 eF450 and human FOXP3 APC Clone PCH101 (eBioscience, Thermo Fischer Scientific)

### Histology

Lung, liver and ear was collected after mice euthanasia and fixed in PFA 4% (Sigma). Tissues section was stained with HE and inflammation was analyzed as described by Workman and al.

### Statistical analysis

Values are represented as means ± SD, unless stated otherwise. GraphPad Prism 6.0 was used for all statistical analyses. P value was calculated with a confidence interval of 95% to indicate the statistical significance between groups. Statistical test included non-parametric Mann-Whitney test, Fischer exact test or two ways ANOVA depending on the dataset. A P value < 0.05 was considered statistically significant. Statistically significant differences between groups are noted in figures with asterisks (*p < 0.05, **p < 0.01, ***p < 0.001, ****p < 0.0001). Correlations were performed with a non-parametric Spearman correlation. Survival was analyzed with Log-rank test (Mantel-Cox).

### Ethics

Animal procedure received our institution ethics committee agreement and Ministère de l'Agriculture agreement according to European directive **2010/63/UE.**

### EXAMPLE 1: PRECLINICAL STUDIES

We established a **scurfy score** based on subscores for each type of clinical symptom: general appearance, behavior, weight loss, degree of desquamation of the tail, blepharitis, crusting of the ears and eczema. Those 7 symptoms do not require any manipulation or sampling and are thus in agreement with the 3R rules. The data are easy to collect and allow to prevent the variability between patients.

**Table 1: Scurfy score of the present disclosure**

| Criteria | Observation | Score |
|---|---|---|
| General apparence | Normal | o |
| | Lack of grooming | 1 |
| | Bristling hair (piloerection) | 2 |
| | Previous observations + crooked back | 3 |
| Behavior | Normal | o |
| | Slightly modified | 1 |
| | Less mobile and isolated but reactive | 2 |
| | Motionless, prostrate, loss of reflexes of alert, reflex pedaling absent | 3 |
| Weight | Value (g) | |
| | Growth/stagnation | o |
| | Loss <10% | 1 |
| | Loss between 10-20% | 2 |
| | Loss >20% | 3 |
| Tail | Normal | o |
| | Focal or moderate desquamation | 1 |
| | Severe desquamation + ring constrictions / loss of part of the tail / Focal inflammation | 2 |
| | Ulceration / loss of the entire tail | 3 |
| Right eye | Normal | 0 |
| | Blepharitis | 1 |
| | Eyes closed | 2 |
| Left eye | Normal | 0 |
| | Blepharitis | 1 |
| | Eyes closed | 2 |
| Right ear | Normal | o |
| | Flattening of the lobes | 1 |
| | Crusting | 2 |
| Left ear | Normal | 0 |
| | Flattening of the lobes | 1 |
| | Crusting | 2 |
| Eczema | Absent | o |
| | Localized | 1 |
| | Diffuse | 2 |
| | Sores / severe lesions scratching | 3 |
| CEdema | 2 legs | 1 |
| | 4 legs | 2 |
| Total | | 24 |

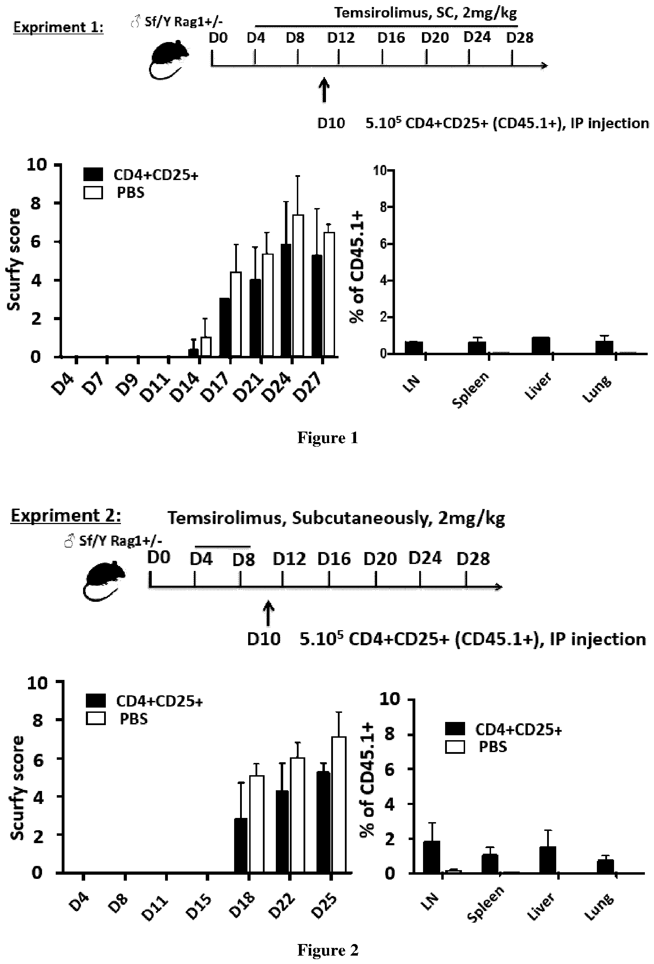

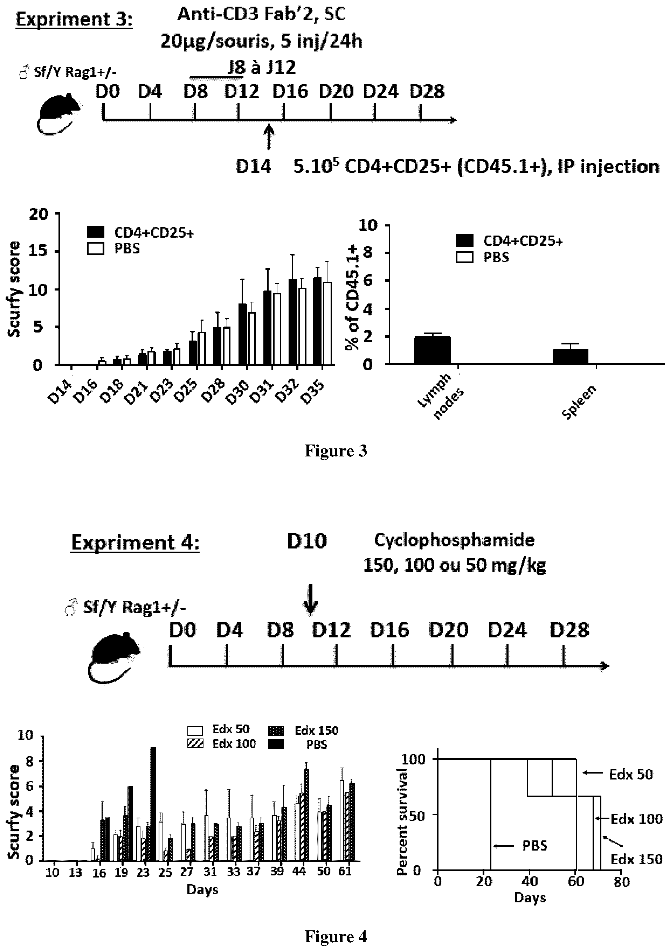

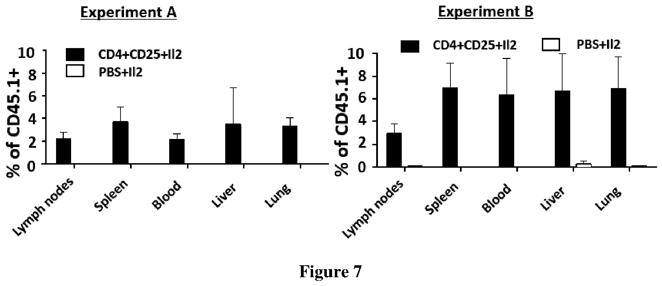

As shown in **Figures 1-7****,** we compared 7 different experimental protocols to identify the one allowing to test the efficacy of Treg to treat Scurfy autoimmune syndrome. **Table 2** summarizes the different experiments. Tregs were sorted on the basis of CD4 and CD25 expression from CD45.1 congenic B6 mice and injected at a dose of 5×10⁵ intra-peritoneally at day 10 or 14. The criteria was the scurfy score measured every 3 to 4 days from birth and when signs of efficiency evidenced improved scores for mice transplanted with Tregs, survival was followed. Three drugs were tested as conditioning regimens: Temsirolimus, subcutaneously injected at a dose of 2mg/kg daily from day 4 to day 28 or from day 4 to day 9 (post-birth), anti-CD3 Fab'2, subcutaneously injected at a dose of 20 micrograms/recipient, daily from day 8 to day 12, Cyclophosphamide at 3 different doses (50, 100 and 150mg/kg) injected at day 10. As shown in **Figures 1-3****,** Temsirolimus and anti-CD3 did not allow to reveal any improvement of Scurfy score after the infusion of Tregs. As shown in Figure 4 cyclophosphamide at all doses delayed the death of scurfy mice to more than 60 days. However, 100 and 150mg/kg led to general toxicity revealed by the health status of the mice. This toxicity was absent at the dose of 50mg/kg. The dose of 50mg/kg led to a more stable scurfy score and allowed to see the benefit of infused Tregs in delaying the worsening of scurfy score and death. The final conditioning regimen thus consists in the injection of 50mg/kg of cyclophosphamide at day 10 before the injection of Tregs at day 14.

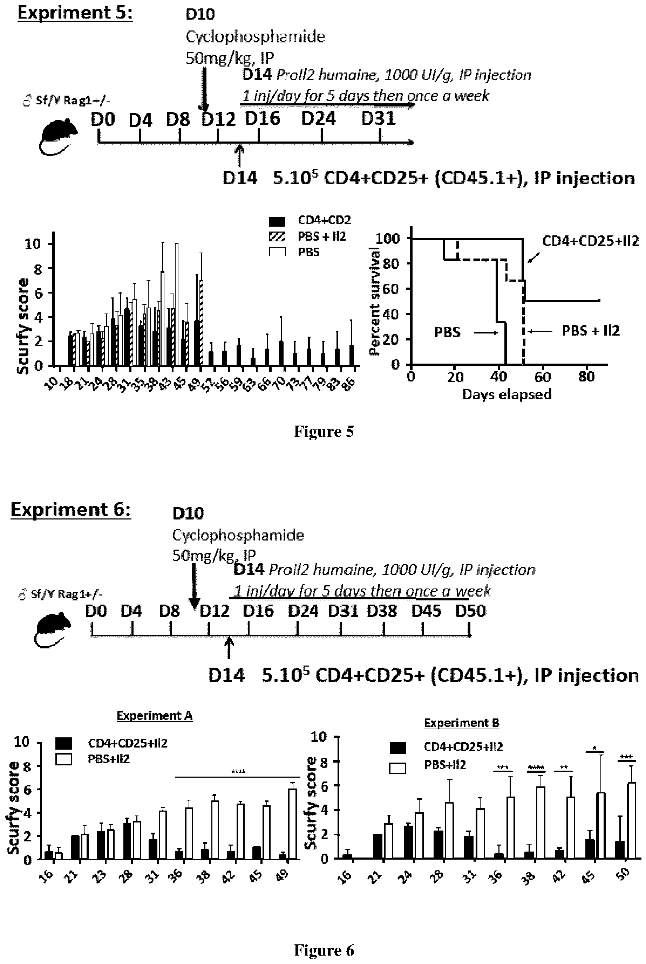

Tregs require IL-2 for their survival (Fontenot, Jason D., et al. "A function for interleukin 2 in Foxp3-expressing regulatory T cells." Nature immunology 6.11 (2005): 1142. And Setoguchi, Ruka, et al. "Homeostatic maintenance of natural Foxp3+ CD25+ CD4+ regulatory T cells by interleukin (IL)-2 and induction of autoimmune disease by IL-2 neutralization." Journal of Experimental Medicine 201.5 (2005): 723-735). Human proleukine 2 was injected intraperitoneally at a dose of 1000UI/g, daily from day 14 to day 18, and once per week thereafter. As shown in **Figure 5****,** following the protocol including IL-2 and cyclophosphamide, T regs delay the death of the patients and are detected in all organs tested, demonstrating that this conditioning regimen allowed their engraftment and persistence in the recipients.

**[Table 2: Summary of the different experiments:]**

| Experiment | Drug | | | | Cell | | | | Maintenance | Read-out |
|---|---|---|---|---|---|---|---|---|---|---|
| | Name | Site of injection | Dose | Delay of injection | Type | Site of injection | Dose | Delay | | |
| 1 | Temsirolimus | Subcutanesously | 2 mg/kg | Twice a week, strating from day 4 | CD4+CD25+ (CD45.1+) | Intraperiteanously | 5,E+05 | Day 10 | Temsirolimus, 2 mg/kg, subcutaneoulsy, twice a week | Scurfy score, weight, cell subset count, histology |
| 2 | Temsirolimus | Subcutanesously | 2 mg/kg | Day 4 and day 8 | CD4+CD25+ (CD45.1+) | Intraperiteanously | S,E+OS | Day 10 | None | Scurfy score, weight, cell subset count, histology |
| 3 | Abti-CD3 Fab'2 | Subcutanesously | 20 ug/mice | Every day from day 4 to day 12 | CD4+CD25+ (CD45.1+) | Intraperiteanously | 5,E+05 | Day 14 | None | Scurfy score, weight, cell subset count, histology |
| 4 | Cyclophosphamide | Intraperiteanously | 50, 100 and 150 mg/kg | Day 10 | CD4+CD25+ (CD45.1+) | None | | | | Scurfy score, weight, survival |
| 5 | Cyclophosphamide | Intraperiteanously | 50 mg/kg | Day 10 | CD4+CD25+ (CD45.1+) | Intraperiteanously | 5,E+05 | Day 10 | Human prolL2, 1000UI/g, intraperitaneously, daily for 5 days after T cell injection, then weekly | Scurfy score, weight, survival |
| 6 A | Cyclophosphamide | Intraperiteanously | 50 mg/kg | Day 10 | CD4+CD25+ (CD45.1+) | Intraperiteanously | 5,E+05 | Day 10 | Human prolL2, 1000UI/g, intraperitaneously, daily for 5 days after T cell injection, then weekly | Scurfy score, weight, cell subset count, histology |
| 6_B | Cyclophosphamide | Intraperiteanously | 50 mg/kg | Day 10 | CD4+CD25+ (CD45.1+) | Intraperiteanously | S,E+OS | Day 10 | Human proIL2, 1000UI/g, intraperitaneously, daily for 5 days after T cell injection, then weekly | Scurfy score, weight, cell subset count, histology |

### EXAMPLE 2

### A specific combination of cyclophosphamide, IL-2 and Tregs cures Scurfy syndrom

Scurfy phenotype included blepharitis, tail and ear eczema and failure to thrive. X^{Sf}/Y.Rag1^{-/+} male mice develop a Scurfy phenotype similar to X^{Sf}/Y.Rag1^{+/+} males with a disease onset at day 8 of life (data not shown). To allow a reproducible evaluation of Scurfy mice phenotype, we developed a specific method of scoring including signs of Scurfy disease (blepharitis, ear and whole-body eczema, tail eczema, limbs edema, body weight, mice appearance and behavior) on a scale from 0 to 21 (Supplemental Table 1). The weight of each criterion in this Scurfy severity score was adjusted depending on the severity of injuries. This method was validated on more than 50 mice and by two independent investigators.

Different immunosuppressive strategies including Temsirolimus (a prodrug of Rapamycine that increases Scurfy life expectancy), anti-CD3 antibody and cyclophosphamide (Cy); were evaluated in order to (i) control Scurfy symptoms, increase mice survival and thus therapeutic window, (ii) mimic IPEX patients conventional immunosuppressive treatments, and (iii) deplete the activated Tconvs compartment to favor engraftment of Tregs. The experimental scheme we used included injection of the immunosuppressive drug, followed by the transplantation of 5.10⁵ congenic CD45.1 WT CD4⁺CD25^{high} Tregs (**Figure 3** and data not shown). Scurfy score was evaluated every two days. Engraftment of WT Treg was quantified in various tissues at study endpoint. Injections of Temsirolimus twice a week starting at disease onset (i.e. at day 8) resulted in reduction of Scurfy score and a doubling of life expectancy as shown by Cheng and coll. (data not shown). However, Scurfy score was not improved by WT Treg transfer at day 10 in accordance with less than 1% chimerism (data not shown). Anti-CD3 Fab'2 injected in a single dose of 20 µg resulted in a nadir of depletion after 5 days and CD4⁺ T cells recovery starting after 10 days (data not shown). Anti-CD3 Fab'2 was injected for 5 consecutive days and Treg were transferred at day 14 of life. Despite a higher engraftment rate (1.9 ± 0.3 % CD45.1⁺ in CD4⁺ T cells in lymph nodes and 1.0 ± 0.4 % CD45.1⁺ in spleen) Scurfy score was not improved by Treg transfer with this anti-CD3 based conditioning regimen (**Figure 3**). Cyclophosphamide (Cy) was administered to Scurfy males at day 10 of life at doses of 50, 100 or 150 mg/mg of body weight. T cells depletion was not different with the three doses. Depletion nadir was observed between day 3 and 5 after Cyclophosphamide injection. Survival was significantly increased following Cy injection from 30 to 90 days as compared to untreated mice (data not shown, p=0.01), allowing a significant increase of the therapeutic window. However, some toxicity was observed including growth retardation and a transient alopecia correlated with Cy doses. Based on these safety and efficacy criteria, we chose the lowest dose of Cy (ie 50 mg/kg) to limit toxicity. Transfer of Treg was performed at day 14 in mice conditioned by a single Cy injection at day 10 (Data not shown). We observed a trend toward a decreased severity score in mice that received Cy and Tregs (Data not shown). However, CD45.1⁺ cells were not detectable at sacrifice in lymph nodes and spleen (Data not shown). On the basis of previous reports showing the beneficial effect of low dose IL-2 on Treg expansion in murine and human models of autoimmunity and transplantation ²⁷⁻²⁹. Scurfy recipients were treated with IL-2 once a day for 5 days then once a week (Data not shown). With the association of Cy, Tregs and IL-2, Scurfy score started to decline after day 28 post-transplantation (p= 0.007) (Data not shown). Then, 49 days after Tregs transfer, CD45.1⁺ chimerism in CD4⁺ T cells reached 2.2% ± 0.6 in lymph nodes, 3.7% ± 1.4 in spleen, 2.1% ± 0.5 in blood, 3.5% ± 3.2 in liver and 3.3% ± 0.8 in lung (Data not shown). Finally, mice life expectancy was increased with a mean survival of 69 days as compared to 39 days in mice that did not receive Tregs (p=0.0004). Interestingly, IL-2 by itself increased survival from a mean survival to 51 days (p=0.03) (Data not shown). Moreover, CD62L staining was increased on CD4 T cells from mice treated with Tregs demonstrating the restoration of a naive CD4 population in lymph nodes (Data not shown).

Thus, the combination of a low dose Cy conditioning with IL-2 treatment post-transplant allowed not only the delay of the disease symptoms, hence increasing the therapeutic window, but also the increase of Tregs engraftment. These results showed for the first time the beneficial effect of Tregs on Scurfy disease after its onset. This optimized murine model was then used to test the suppressive functionality of gene corrected scurfy CD4 T cells.

### Engineered Scurfy CD4 T cells with LNGFR.FOXP3 vector rescue Scurfy mice after the onset of the disease

To test the ability of of CD4^{LNGFR.FOXP3} required to control Scurfy disease, we treated X^{Sf}/Y.Rag1^{-/+} male with one I.P injection of Cy at day 10 as previously described followed by an injection of congenic 5.10⁵ CD45.1 Tregs or 5.10⁵, 7.5.10⁵ or 1.10⁶ scurfy CD4^{LNGFR.FOXP3} at 14 days of life. Follow-up of Scurfy score showed a dose-dependent inhibition of Scurfy symptoms according to the number of injected CD4^{LNGFR.FOXP3} (ANOVA test, p-value= 0.0039, Data not shown). Moreover, after 50 days of follow-up, injection of 7.5.10⁵ CD4^{LNGFR.FOXP3} demonstrated similar results in term of Scurfy score but also percentage of chimerism as compared to 5.10⁵ Tregs (Data not shown). Therefore, the dose of 7.5.10⁵ CD4^{LNGFR.FOXP3} was selected for further evaluation. Surprisingly, CD62L staining was not different in the three doses of CD4^{LNGFR.FOXP3} (Data not shown).

Then, in order to compare CD4^{LNGFR.FOXP3} efficacy to Tregs, adoptive transfer of CD45.1 WT Tregs, CD4^{LNGFR.FOXP3} and its mock counterpart, CD4^{LNGFR} or vehicle (PBS) was performed. Mice were carefully followed until their sacrifice at day 50 of life. To note, VCN were similar between the three vectors (1.3 for LNGFRp-eFOXP3, 1.2 for LNGFRp-e and 1.5 for LNGFRe-p vectors). Scurfy score increased similarly in all groups up to day 27 (Data not shown). At day 32, while it continues to rise in the same way in mice that received Cy and IL-2 alone or with CD4^{LNGFR}, we observed a significant decrease in mice treated with WT Tregs and CD4^{LNGFR.FOXP3} (p-value=0.007 and 0.0008 respectively). More specifically, WT Tregs and CD4^{LNGFR.FOXP3} treated mice recovered of alopecia induced by Cy, presented a mild eczema of the tail, low level of blepharitis and gained weight whereas diluent and CD4^{LNGFR}treated mice presented failure to thrive and severe eczema of the whole body (Data not shown).

Analysis of chimerism showed a mean percentage of CD45.1 Treg of 5.0 ranging from 1.7 to 12.6% in lymph nodes, spleen, blood, liver and lung CD4 T cells (Data not shown). ΔLNGFR⁺ chimerism in mice treated with CD4^{LNGFR.FOXP3} T cells were slightly lower with a mean of 3.4% ranging from 0.2 to 4.6% in lymph nodes, spleen, blood, liver and lung (Data not shown). On the opposite, CD4^{LNGFR} T cells did not expand and percentage remained inferior to 1.5% in all tissues. Importantly, human FOXP3 expression persisted in ΔLNGFR⁺ CD4⁺ collected from lymph nodes of CD4^{LNGFR.FOXP3} treated mice (Data not shown). ΔLNGFR⁺ cells were sorted from lymph nodes lymphocytes and VCN analysis were quantified at 2.3±0.8 for LNFGR.FOXP3 vector and 1.6±0.7 for LNGFR vector (Data not shown).

Analysis of CD62L staining in lymph nodes illustrated in Figure 4G demonstrated that a subset of naive CD4⁺ T cells was restored in mice treated with Tregs and CD4^{LNGFR.FOXP3} cells. CD4⁺ T cells in lymph nodes contained 15.7±0.6% of CD62L⁺ cells in mice treated by Cy and IL-2 against 78.1±2.4% in WT mice. Treatment with Tregs increased this level to 44.0±6.2% and with Scurfy CD4^{LNGFR.FOXP3} T cells to 31.1±11.8%, as compared to 20.8±2.5 CD62L⁺ T CD4 after transplantation of CD4^{LNGFR} T cells. Histology analysis showed no significant difference in the inflammation score in the lung, liver and skin (data not shown).

Survival was significantly increased following Cy+IL-2+CD4^{LNGFR.FOXP3} treatment as compared to Cy treated Scurfy mice with respectively a mean survival of 64 days to 47 days (p= 0.0195). Similarly, mean survival was significantly increased with Tregs as compared to Cy with a survival above 89 days (p-value<0.0001). Mean survival for Cy+IL-2+Treg treated mice was not significantly different as compared to Cy+IL-2+ CD4^{LNGFR.FOXP3} treated mice (p=0.1047). Survival was not different between Cy+IL-2+PBS and Cy+IL-2+CD4^{LNGFR} treated mice with a mean survival of respectively of 54.5 days and 53 day (p=0.8729) (Figure 3H). After 90 days of follow-up, CD45.1 Tregs and CD4^{LNGFR.FOXP3} chimerism in CD4⁺ T cells decreased as compared to day 50 analyses with a mean percentage of 1.5% and 1.1% respectively. Importantly, hFOXP3 was still detectable in CD4^{LNGFR.FOXP3} demonstrating the stability of hFOXP3 in transduced CD4⁺ T cells *in vivo.*

These results demonstrated that FOXP3 expression in Scurfy CD4⁺ T cells recapitulated a suppressive function and transduced CD4^{LNGFR.FOXP3} were able to rescue the severe Scurfy autoimmune disease.

### CONCLUSION:

Transfer of splenocytes or sorted Tregs in Scurfy deficient mice has been shown to prevent Scurfy symptoms when transferred within the two first days of live. In this study, we demonstrated that Treg transfer at day 14 of life allowed long-term rescue of Scurfy symptoms if combined with Cy conditioning followed by low dose of IL-2 injections. This was demonstrated with robust parameters including a clinical score, staining of CD4⁺ T cells, analysis of chimerism and survival. In the different strategies tested for Treg adoptive transfer, Cy allowed the best control of autoimmunity. Cy has been shown to deplete the T cell niche in mice. CD4 T cells nadir was obtained 4 days after injection and cell count normalized at 10 days. Moreover, Cy allows a functional impairment of activated T cells resulting in a relative enrichment in Tregs. However, in young animals, even low dose of Cy resulted in toxicities as alopecia and growth retardation. Other conditioning to tip the balance between Tregs and Tconvs based on a more specific depletion of activated T cells would be a high requirement for clinical application. For example, non-mitogenic anti-CD3 antibodies could be interesting. However, in our Scurfy mice model, we were not able to demonstrate its efficacy to allow an appropriated engraftment of Tregs. Then, low dose IL-2 has been shown to favor Treg expansion and thus has beneficial therapeutic effects in the context of several autoimmune diseases such as type I diabetes, systemic lupus erythematous and others. IL-2 also enhances proliferation of donor-specific Tregs and promotes tolerance in allogeneic transplantation. In 10 weeks-old NOD mice, it has been demonstrated that daily injection of 25.000UI/day during five day was able to reverse diabetes. In order to adapt this low dosing of IL-2 to 14 days-old Scurfy mice, we decided to use 1.000UI/g of mice. Moreover, based on the TRANSREG study, this induction therapy was completed by a maintenance therapy of weekly IL-2 injections. Importantly, treatment with low dose IL-2 did not worsen auto-immunity in Scurfy settings. The remaining question would concern the end of IL-2 treatment. Altogether, this therapeutic strategy allowed evaluating cells suppressive activity in the *in vivo* Scurfy model of autoimmunity.

In these settings, Scurfy CD4 T cells transduced with LNGFR.FOXP3 vector were able to rescue Scurfy disease, demonstrating the efficiency of the lentiviral vector to induce regulatory functions with a mean of 2 VCN per cell. This suggested that the homology between human and murine *FOXP3* allows the expression of human *FOXP3* in murine CD4⁺ T cells by itself to be sufficient to induce suppressive function to CD4⁺ cells. Interestingly Scurfy CD4⁺ T cells transduced with LNGFR.FOXP3 vector expanded preferentially as compared to Scurfy CD4⁺ T transduced with the mock LNGFR vector. This could be explained by their increased sensibility to IL-2 due to higher level of CD25. Moreover, these adoptively transferred Tregs were stably maintained until 90 days *in vivo* in an inflammatory context, and expressed durably FOXP3. In our assay, transfer of *bona fide* Tregs allowed a slightly better control of Scurfy disease as compared to genetically engineered CD4⁺ T cells. Several factors could explain those differences. First, the level of chimerism was half the one of WT Tregs at day 50 and also in long-term follow-up at day 90. Consequently, increasing the cell dose or recurrent infusion could improve outcome. Then, our vector allowed the expression of human FOXP3 protein, which present 86% of homology with murine FOXP3 and may be do not fully recapitulate Tregs transcriptomic program. Moreover, engineered regulatory CD4^{LNGFR.FOXP3} were collected from Scurfy mice and cultured for 5 days. Consequently, they might be more sensitive to sorting and manipulation. Beyond, preselecting naïve T cell might result in more efficient suppressive cells as demonstrated by Passerini and al. CD4^{LNGFR.FOXP3}. In our work, after adoptive transfer of Treg or CD4^{LNGFR.FOXP3} some Scurfy mice died mostly of Scurfy symptoms recurrence. Therefore, multiple injections of Tregs or increasing of IL-2 dose or frequencies of injection could be considered to improve Scurfy disease control.

### REFERENCES:

Throughout this application, various references describe the state of the art to which this disclosure pertains.

## Claims

1. An amount of cyclophosphamide, an amount of a population of Treg cells and an amount of a IL-2 polypeptide for use in a method of inducing or restoring immune tolerance in a patient suffering from IPEX syndrome comprising the steps of i) administering the patient with the said amount of cyclophosphamide, ii) then engrafting the patient with the said amount of the population of Treg cells, and iii) finally administering the patient with the said amount of a IL-2 polypeptide.

2. The amount of cyclophosphamide, the amount of the population of Treg cells and the amount of a IL-2 polypeptide for use according to claim 1 wherein the said amount of cyclophosphamide of between 40 à 200 mg/m² is used, preferably an amount of 150 mg/m² is used.

3. The amount of cyclophosphamide, the amount of the population of Treg cells and the amount of a IL-2 polypeptide for use according to claim 1 wherein the amount of cyclophosphamide is administered to the patient in one bolus 2, 3, 4, 5, 6, 7, 8, 9 or 10 days before engrafting the patient with the amount of Treg cells.

4. The amount of cyclophosphamide, the amount of the population of Treg cells and the amount of a IL-2 polypeptide for use according to claim 1 wherein the Treg cells are prepared by transfecting or transducing a population of T cells ex vivo with a vector comprising a nucleic acid encoding for FoxP3.

5. The amount of cyclophosphamide, the amount of the population of Treg cells and the amount of a IL-2 polypeptide for use according to claim 1 wherein the Treg cells are prepared by a gene editing approach for site- specific restoration of wild-type FOXP3 gene expression applied to T cells and/or hematopoietic stem cells (HSPCs) and/or T-cell progenitors carrying FOXP3 mutations to correct Treg functional defects.

6. The amount of cyclophosphamide, the amount of the population of Treg cells and the amount of a IL-2 polypeptide for use according to claim 1 wherein the Treg cells are genetically modified for expressing a chimeric antigen receptor (CAR).

7. The amount of cyclophosphamide, the amount of the population of Treg cells and the amount of a IL-2 polypeptide for use according to claim 1 wherein an amount of between 1×10⁶/kg and 10×10⁶/kg Treg cells is engrafted in the patient.

8. The amount of cyclophosphamide, the amount of the population of Treg cells and the amount of a IL-2 polypeptide for use according to claim 1 wherein the IL-2 polypeptide is a IL-2 mutein.

9. The amount of cyclophosphamide, the amount of the population of Treg cells and the amount of a IL-2 polypeptide for use according to claim 1 wherein an amount of the IL-2 polypeptide of between 0,5 MUI (Million International Units) / day and 1,5 million of MUI/day is used.

10. The amount of cyclophosphamide, the amount of the population of Treg cells and the amount of a IL-2 polypeptide for use according to claim 1 wherein the IL-2 polypeptide is administered to the patient daily from day 1 to day 5 (the induction period) after engraftment, and then every 2 weeks from day 15 to day 180 (the maintenance period) after engraftment.

## Patentansprüche

1. Menge an Cyclophosphamid, Menge einer Population von Treg-Zellen und Menge eines IL-2-Polypeptids zur Verwendung bei einem Verfahren zum Induzieren oder Wiederherstellen von Immuntoleranz bei einem Patienten, der am IPEX-Syndrom leidet, umfassend die Schritte: i) Verabreichen der Menge an Cyclophosphamid an den Patienten, ii) anschließend Transplantieren der Menge der Population von Treg-Zellen in den Patienten, und iii) schließlich Verabreichen der Menge eines IL-2-Polypeptids an den Patienten.

2. Menge an Cyclophosphamid, Menge der Population von Treg-Zellen und Menge eines IL-2-Polypeptids zur Verwendung nach Anspruch 1, wobei die Menge an Cyclophosphamid zwischen 40 und 200 mg/m² verwendet wird, vorzugsweise eine Menge von 150 mg/m² verwendet wird.

3. Menge an Cyclophosphamid, Menge der Population von Treg-Zellen und Menge eines IL-2-Polypeptids zur Verwendung nach Anspruch 1, wobei die Menge an Cyclophosphamid dem Patienten in einem Bolus 2, 3, 4, 5, 6, 7, 8, 9 oder 10 Tage vor dem Transplantieren der Menge an Treg-Zellen in den Patienten verabreicht wird.

4. Menge an Cyclophosphamid, Menge der Population von Treg-Zellen und Menge eines IL-2-Polypeptids zur Verwendung nach Anspruch 1, wobei die Treg-Zellen durch Transfektion oder Transduktion einer Population von T-Zellen ex vivo mit einem Vektor hergestellt werden, der eine für FoxP3 codierende Nukleinsäure umfasst.

5. Menge an Cyclophosphamid, Menge der Population von Treg-Zellen und Menge eines IL-2-Polypeptids zur Verwendung nach Anspruch 1, wobei die Treg-Zellen durch einen Geneditierungsansatz zur ortsspezifischen Wiederherstellung der Wildtyp-FOXP3-Genexpression hergestellt werden, die auf T-Zellen und/oder hämatopoetische Stammzellen (HSPCs) und/oder T-Zell-Vorläufer, die FOXP3-Mutationen tragen, angewendet wird, um Treg-Funktionsdefekte zu korrigieren.

6. Menge an Cyclophosphamid, Menge der Population von Treg-Zellen und Menge eines IL-2-Polypeptids zur Verwendung nach Anspruch 1, wobei die Treg-Zellen genetisch modifiziert sind, um einen chimären Antigenrezeptor (CAR) zu exprimieren.

7. Menge an Cyclophosphamid, Menge der Population von Treg-Zellen und Menge eines IL-2-Polypeptids zur Verwendung nach Anspruch 1, wobei eine Menge zwischen 1^{∗}10⁶/kg und 10^{∗}10⁶/kg Treg-Zellen in den Patienten transplantiert wird.

8. Menge an Cyclophosphamid, Menge der Population von Treg-Zellen und Menge eines IL-2-Polypeptids zur Verwendung nach Anspruch 1, wobei das IL-2-Polypeptid ein IL-2-Mutein ist.

9. Menge an Cyclophosphamid, Menge der Population von Treg-Zellen und Menge eines IL-2-Polypeptids zur Verwendung nach Anspruch 1, wobei eine Menge des IL-2-Polypeptids zwischen 0,5 MIE (Millionen Internationale Einheit)/Tag und 1,5 Millionen von MIE/Tag verwendet wird.

10. Menge an Cyclophosphamid, Menge der Population von Treg-Zellen und Menge eines IL-2-Polypeptids zur Verwendung nach Anspruch 1, wobei das IL-2-Polypeptid dem Patienten täglich von Tag 1 bis Tag 5 (die Induktionsperiode) nach der Transplantation und dann alle 2 Wochen von Tag 15 bis Tag 180 (die Erhaltungsperiode) nach der Transplantation verabreicht wird.

## Revendications

1. Quantité de cyclophosphamide, quantité d'une population de cellules Treg et quantité d'un polypeptide IL-2 pour une utilisation dans un procédé d'induction ou de restauration d'une tolérance immunitaire chez un patient souffrant du syndrome IPEX comprenant les étapes consistant à i) administrer au patient ladite quantité de cyclophosphamide, ensuite ii) implanter dans le patient ladite quantité de la population de cellules Treg, et finalement iii) administrer au patient ladite quantité d'un polypeptide IL-2.

2. Quantité de cyclophosphamide, quantité de la population de cellules Treg et quantité d'un polypeptide IL-2 pour une utilisation selon la revendication 1, dans laquelle ladite quantité de cyclophosphamide entre 40 et 200 mg/m² est utilisée, de préférence une quantité de 150 mg/m² est utilisée.

3. Quantité de cyclophosphamide, quantité de la population de cellules Treg et quantité d'un polypeptide IL-2 pour une utilisation selon la revendication 1, dans laquelle la quantité de cyclophosphamide est administrée au patient dans un bolus 2, 3, 4, 5, 6 , 7, 8, 9 ou 10 jours avant d'implanter dans le patient la quantité de cellules Treg.

4. Quantité de cyclophosphamide, quantité de la population de cellules Treg et quantité d'un polypeptide IL-2 pour une utilisation selon la revendication 1, dans laquelle les cellules Treg sont préparées par transfection ou transduction d'une population de cellules T ex vivo avec un vecteur comprenant un acide nucléique codant pour FoxP3.

5. Quantité de cyclophosphamide, quantité de la population de cellules Treg et quantité d'un polypeptide IL-2 pour une utilisation selon la revendication 1, dans laquelle les cellules Treg sont préparées par une approche d'édition génique pour la restauration spécifique au site de l'expression du gène FOXP3 de type sauvage appliquée à des cellules T et/ou à des cellules souches hématopoïétiques (HSPC) et/ou à des progéniteurs de cellules T porteurs de mutations FOXP3 pour corriger les défauts fonctionnels des Treg.

6. Quantité de cyclophosphamide, quantité de la population de cellules Treg et quantité d'un polypeptide IL-2 pour une utilisation selon la revendication 1, dans laquelle les cellules Treg sont génétiquement modifiées pour exprimer un récepteur d'antigène chimère (CAR).

7. Quantité de cyclophosphamide, quantité de la population de cellules Treg et quantité d'un polypeptide IL-2 pour une utilisation selon la revendication 1, dans laquelle une quantité entre 1^{∗}10⁶/kg et 10^{∗}10⁶/kg de cellules Treg est implantée dans le patient.

8. Quantité de cyclophosphamide, quantité de la population de cellules Treg et quantité d'un polypeptide IL-2 pour une utilisation selon la revendication 1, dans laquelle le polypeptide IL-2 est une mutéine IL-2.

9. Quantité de cyclophosphamide, quantité de la population de cellules Treg et quantité d'un polypeptide IL-2 pour une utilisation selon la revendication 1, dans laquelle une quantité du polypeptide IL-2 entre 0,5 MUI (Million d'Unités Internationales)/jour et 1,5 millions de MUI/jour est utilisée.

10. Quantité de cyclophosphamide, quantité de la population de cellules Treg et quantité d'un polypeptide IL-2 pour une utilisation selon la revendication 1, dans laquelle le polypeptide IL-2 est administré quotidiennement au patient du jour 1 au jour 5 (la période d'induction ) après l'implantation, et ensuite toutes les 2 semaines du jour 15 au jour 180 (la période d'entretien) après l'implantation.
